# EUROPEAN PATENT APPLICATION

(11) **EP 1 627 707 A1**
(43) Date of publication of application: **22.02.2006**
(21) Application number: 03730625.5
(22) Date of filing: 26.05.2003
(51) Int. Cl.: B25B 9/00, B25J 7/00, B25J 15/08

(54) **ELECTRIC TWEEZERS**

(71) Applicant: MITSUBISHI DENKI KABUSHIKI KAISHA, Tokyo 100-8310 (JP)
(72) Inventor: MURAMATSU, Naoki, c/o Mitsubishi Denki K.K., Chiyoda-ku, Tokyo 100-8310 (JP)
(74) Representative: HOFFMANN EITLE
(86) International application number: PCT/JP2003/006516
(87) International publication number: WO 2004/103647

(57) **Abstract**

Electric tweezers 1 includes: a case 60 for housing a motor 52; a finger 13, one end portion thereof being connected and fixed to the case 60, and the other end portion 13g thereof being connected to the shifting element, for magnifying the amount by which the tips open/close based on the translational displacement of the shifting element, and for thereby releasing/holding an object; a switch 57 for starting/stopping the motor rotation of the motor 52; and a conversion mechanism 30 that is housed in the case 60 and connected to a shaft 52a of the motor 52, and converts unidirectional rotation of the motor 52 into predefined reciprocating translational displacement with respect to the other end portion 13g.

## Description

### TECHNICAL FIELD

The present invention relates to an improvement of tweezers for holding a minute part, and particularly relates to simple electric tweezers having a displacement magnifying mechanism.

### BACKGROUND ART

The inventor of the invention proposed a gripper having a very simple mechanism in PCT Gazette Publication No. W099/3087. The gripper has a characteristic in which a small translational shifting amount is magnified about 10 times at the tip of a finger and then converted into a bending displacement amount, by applying a buckling phenomenon of a long pole to the finger (holding member), without having any particular displacement magnifying mechanism.

These grippers described above, because the gripper can softly grasp a minute part and is small and light, have been suitable for a robot arm.

As the result of repeated enthusiastic researches and investigations, the inventor has discovered the following technical problem, and, resultantly, made an application for the invention related to electric tweezers in which the problem has been resolved. That is, if the gripper is used for holding an object by a predefined opening operation and closing (holding) operation, an opening amount and closing amount of a holding member must be set at an appropriate value. Therefore, motor rotational angles in the normal direction and the reverse direction must be adjusted such that a translational displacement amount in a reciprocating motion at the other end portion of a holding member comes to a predefined value, which results in the gripper being complicated. The inventor applied the invention (PCT Publication No. PCT/JP01/10324) related to electric tweezers, in which the above complication has been significantly diminished.

However, because the small translational shifting amount is converted into a bending displacement magnified about 10 times at the tip of a finger, a fine adjustment must be consequently needed for connecting and fixing one end portion of the finger to a case, or for connecting and fixing the other end portion to a translational shifting element. Moreover, it must be needed to adjust an opening/closing amount of the holding member according to a held object.

### DISCLOSURE OF THE INVENTION

The present invention has been made in order to solve above problem, and to provide electric tweezers capable of easily mounting and adjusting a holding member having a magnifying function of a translational displacement, and capable of easily adjusting an opening/closing amount of the holding member.

Electric tweezers relating to a first aspect of the present invention comprises: a motor having a shaft; a conversion mechanism connected to the shaft, having a shifting element that in shifting converts unidirectional rotation of the shaft into predefined reciprocating translational displacement with respect to the shaft; a case for housing the conversion mechanism and the motor; a holding member, one end portion thereof being connected and fixed to the case, and the other end portion thereof being connected to the shifting element, for magnifying the amount by which the tweezer tips open/close based on the translational displacement of the shifting element, and for thereby releasing/holding an object; a switching means for starting/stopping the motor rotation; an adjusting member, one end facet thereof being connected either to the one end portion or the other end portion of the holding member, and the other end facet thereof being connected either to the shifting element or the case, so as to be elastically deformed; and a pressuring means for pressuring the adjusting member a predefined amount, so as to regulate the separation between the one end portion and the other end portion in the translational displacement direction.

The electric tweezers described in above explanation execute a first operation that the converting mechanism converts unidirectional rotation of the motor into predefined reciprocating translational displacement with respect to the other end portion of the holding member, and the switching means rotates and stops the motor. That is, the conversion mechanism converts the translational displacement of the other end portion of the holding member into a predefined value such that the opening/closing amount of the holding member becomes constant. Therefore, the present invention can bring a first effect of easily obtaining electric tweezers in which, for example, a certain object can be closed (held) or released.

Moreover, the pressuring means pressures the elastic member a predefined amount so as to adjust a distance in a motor shaft direction between one end portion and the other end portion of the holding member component, in other words, the means adjusts a distance in a translational displacement direction of the shifting element. Therefore, the present invention can bring an effect in that the opening/closing amount of the holding member can be easily adjusted, and the holding member can be easily adjusted to mount.

Electric tweezers relating to a second aspect of the invention comprises: a motor having a shaft; a conversion mechanism connected to the shaft, having a shifting element that in shifting converts unidirectional rotation of the shaft into predefined reciprocating translational displacement with respect to the shaft; a case for housing the conversion mechanism, the case being divided into at least first and second case members, each member being threaded and the second case member are screwing into the first case member; a holding member, one end portion thereof being connected and fixed to the first case member, and the other end portion thereof being connected to the shifting element of the conversion mechanism, for magnifying the amount by which, based on the translational displacement of the shifting element, the tweezer tips open and close, and for thereby releasing/holding an object; a switching means for starting/stopping the motor rotation; and a first elastic member having a hollow portion, the member being placed between the first case member and second case member, so as to be elastically deformed.

The electric tweezers described in above explanation can bring the first operation and effect, in addition, the opening/closing amount of the holding member can easily be adjusted by adjusting a distance of one end portion in a translational displacement direction with respect to the other end portion of the holding member by adjusting a screwed amount between the first case member and the second case member so as to deform the first elastic member. That is, if the first elastic component is contracted by a screwed amount between the first case member and the second case member, an opening amount at the tip of the holding member is increased; on the contrary, if the first elastic member is expanded, the opening amount at the tip of the holding member is decreased. Therefore, the present invention can bring an effect in that the opening/closing amount of the holding member can be easily adjusted, and the holding member can be easily adjusted to mount.

Electric tweezers relating to a third aspect of the invention comprises: a motor having a shaft; a case for housing the motor; a holding member, one end portion thereof being connected and fixed to the case, and the other end portion thereof for magnifying the amount by which, based on reciprocating translational displacement, the tweezer tips open/close, and for thereby releasing/holding an object; a switching means for starting/stopping the motor rotation; a conversion mechanism, connected to the shaft, having a screw hole at one end of a shifting element that in shifting converts unidirectional rotation of the motor into predefined reciprocating translational displacement with respect to the other end of the holding member; a screw member screwed to the screw hole; and a second elastic member having a hollow portion, the member being placed between the screw member and the shifting element, so as to be elastically deformed.

The electric tweezers described in above explanation can bring the first operation and effect; in addition, if the second elastic member is contracted by screwing the screw member into the screw hole, an opening amount at the tip of the holding member is decreased; on the contrary, if the second elastic member is magnified, the opening amount at the tip of the holding member is increased. Therefore, the present invention can bring an effect in that the opening/closing amount of the holding member can be easily adjusted, and the holding member can be easily adjusted to mount.

Electric tweezers relating to a fourth aspect of the invention, wherein the conversion mechanism is connected to the shaft and comprises: a cam having a lift portion; a cam follower, connected to the other end portion of the holding member, for converting unidirectional rotation of the cam into predefined reciprocating translational displacement with respect to the other end portion, and having an abutting portion that abuts on the lift portion; and a pressuring member housed in the case, for abutting, by the cam or the cam follower being pressured, the lift portion on the abutting portion.

The electric tweezers described in above explanation can bring an effect of obtaining conversion mechanism that converts unidirectional rotation of the motor into predefined reciprocating translational displacement with respect to the other end portion, and ensuring the transmission of a cam action by the motor rotation to the cam follower, because both the cam and the cam follower are pressured by the pressuring member.

Electric tweezers relating to a fifth aspect of the invention is
characterized in that: the pressuring member and the cam follower are housed in the second case member; and the other end portion of the holding member is fixed on the first case member.

The electric tweezers described in above explanation can realize the electric tweezers in which a deformation of the first elastic member does not affect to the contraction/expansion of the pressuring member, and a holding force is kept on a constant value with adjusting opening/closing amounts of the holding member.

Electric tweezers relating to a sixth aspect of the invention comprises: a motor having a shaft; a conversion mechanism connected to the shaft, having a shifting element that in shifting converts unidirectional rotation of the shaft into predefined reciprocating translational displacement with respect to the shaft; a holding member, having one end portion, the other end portion thereof being connected to the shifting element, for magnifying the amount by which, based on the translational displacement amount of the shifting element the tweezer tips open/close, and for thereby releasing/holding an object; a base member having an engaging portion for engaging with the other end portion of the holder; a finger holder having a hole, and a concave portion for engaging the base member attachably/detachably, the inside face of the concave portion being threaded; a switching means for starting/stopping the motor rotation; a conversion mechanism connected to the shaft, for converting a unidirectional rotation of the motor into predefined reciprocating translational displacement with respect to a shared portion of the holding member; a third elastic member, having a hole, placed between the base member and the finger holder; and a threaded nut member for screwing to the thread of the finger holder.

The electric tweezers described in the above explanation can bring the first operation and effect; in addition, if the third elastic member is contracted by screwing the nut member into the screw hole, an opening amount at the tip of the holding member is decreased; on the contrary, if the third elastic member is magnified, the opening amount at the tip of the grasping component is increased. Therefore, the present invention can bring an effect in that the opening/closing amount of the holding member can be easily adjusted, and the holding member component can be easily adjusted to mount.

### BRIEF DESCRIPTION OF DRAWINGS

Fig. 1 is an entire cross-sectional view of electric tweezers according to Embodiment 1 of the invention;
Fig. 2 is an oblique perspective view illustrating a finger unit used for the electric tweezers illustrated in Fig. 1;
Figs. 3 are views illustrating a cam mechanism illustrated in Fig. 1, in which an elevation view "a" of the cam follower, a right side view "b" of the cam follower, a left side view "c" of the cam follower, and an elevation view "d" of the cam are illustrated;
Figs. 4 are views including a left side view "a", an elevation view "b", and a right side view "c" of a finger holder used for in the electric tweezers illustrated in Fig. 1;
Figs. 5 are views including a left side view "a" and an elevation view "b" of a finger holder used for the electric tweezers according to another Embodiment of the invention;
Figs. 6 are views including a left side view "a" and an elevation view "b" of a cam follower holder used for the electric tweezers according to another Embodiment of the invention;
Fig. 7 is a cross-sectional view of an assembly of the finger holder illustrated in Fig. 5 and the cam follower holder illustrated in Fig. 6;
Figs. 8 are exploded oblique perspective views of a opening/closing amount adjusting mechanism of the holding finger of the electric tweezers according to another Embodiment of the invention;
Figs. 9 are views including a left side view "a" and an elevation view "b" in which a fixing screw and an elastic washer illustrated in Fig. 8 are assembled;
Figs. 10 are exploded perspective views of an opening/closing amount adjusting mechanism of the holding finger of the electric tweezers according to another Embodiment of the invention;
Figs. 11 are views including a left side view "a", an elevation view "b", and a right side view "c" of a finger holder used for the finger holder illustrated in Fig. 10;
Fig. 12 is a plane view illustrating a finger base illustrated in Fig. 10; and
Fig. 13 is a cross-sectional view of an assembly of the finger holder illustrated in Fig. 11 and the finger base, etc., illustrated in Fig. 12.

### BEST MODE FOR CARRYING OUT THE INVENTION

### Embodiment 1

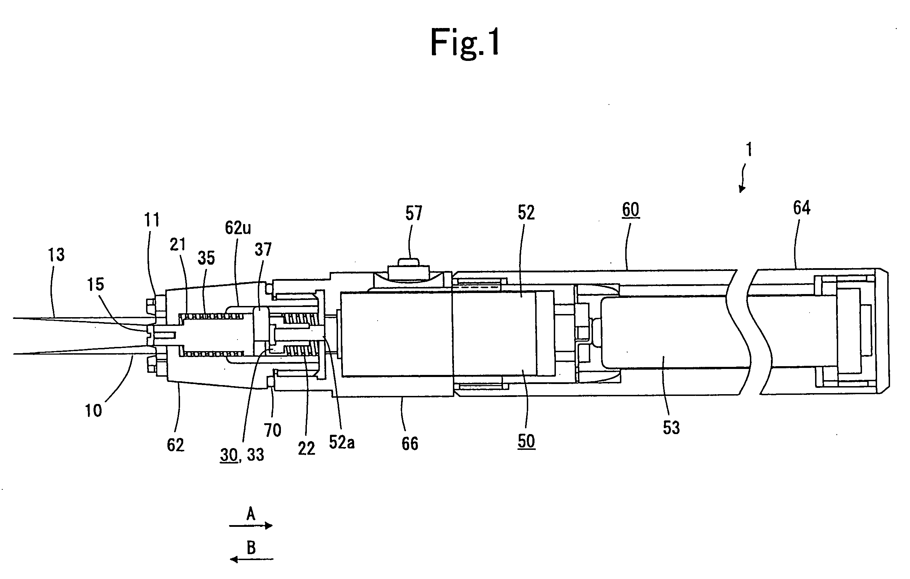

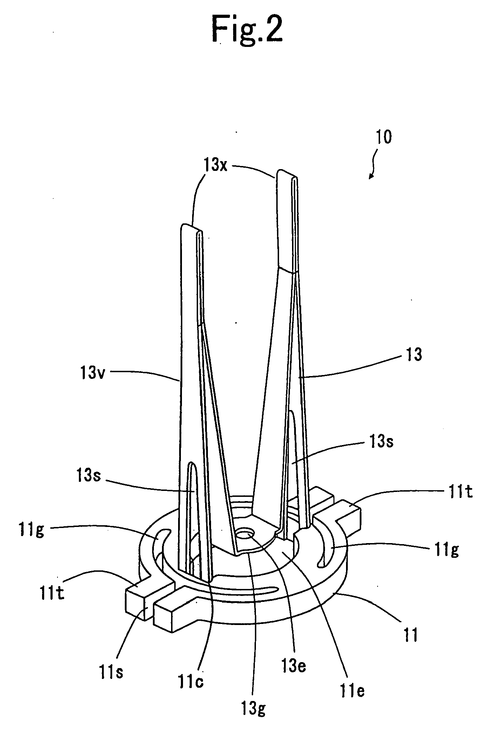

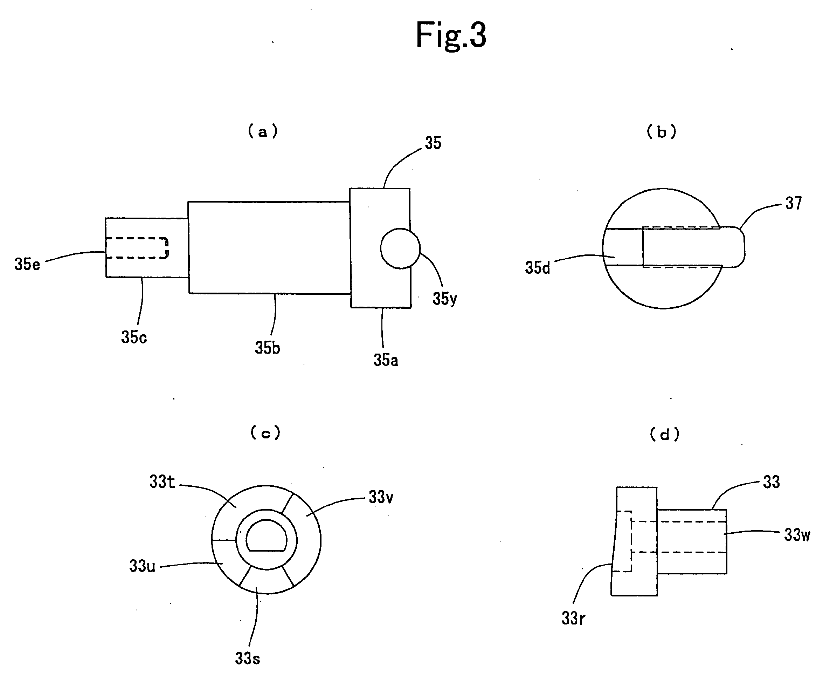

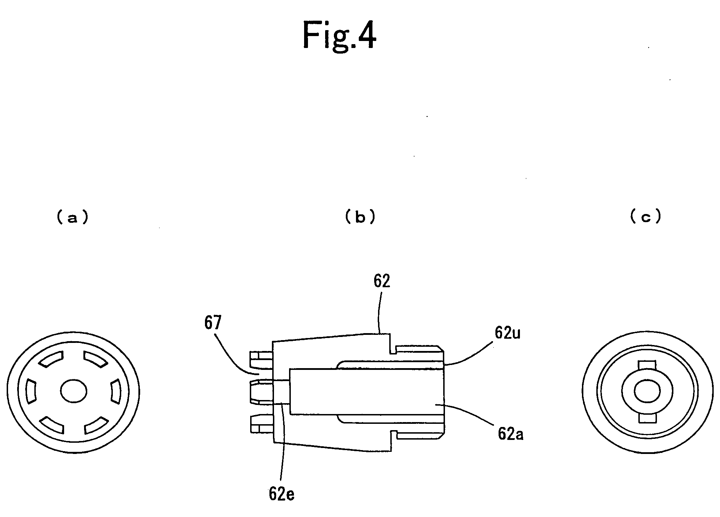

Electric tweezers in Embodiment 1 of the invention will be explained using Fig. 1 through Fig. 4. Fig. 1 is an entire cross-sectional view of the electric tweezers according to Embodiment 1 of the invention; Fig. 2 is an oblique perspective view illustrating a finger unit used for the electric tweezers illustrated in Fig. 1; Figs. 3 are views illustrating a cam mechanism illustrated in Fig. 1, in which an elevation view "a" of the cam follower, a right side view "b" of the cam follower, a left side view "c" of the cam follower, and an elevation view "d" of the cam are illustrated; and Figs. 4 are views including a left side view "a", an elevation view "b", and a right side view "c" of a finger holder used for the electric tweezers illustrated in Fig. 1.

In Fig. 1, electric tweezers "1" comprises: a holding finger "13" as a holding member having one end portion and the other end portion; a finger unit "10"; a driving unit "50" that is connected to a shared portion (the other end portion) "13g" of the holding finger 13, and has a motor "52" for translationally displacing the shared portion 13; a conversion mechanism "30" for converting unidirectional rotation, i.e., a predefined normal rotation or a reverse rotation, of an shaft "52a" of the motor 52 into predefined reciprocating translational displacement, in which the forward displacement is equal to the backward displacement, with respect to the shared portion 13g of the holding finger 13; a fixing screw "15" for fixing the shared portion 13g of the holding finger 13 to one end portion of the conversion mechanism 30; and a case "60" for housing the driving unit 50 and the conversion mechanism 30 and fixing the finger unit 10, and having a function of adjusting an opening/closing amount of the holding finger 13.

The finger unit 10 is comprised of the holding finger 13 as the holding member and a finger base "11" as a base member. The holding finger 13 comprises: the shared portion 13g, as the other end portion, having a hole "13e" at the center thereof; slits "13s" are provided outside V-shaped sections "13v" at the center thereof; and the two V-shaped sections 13v, being roughly V-shaped, allow the sections to open/close according to a magnifying displacement of tips "13x" (to the horizontal directions in Fig. 1) by pulling or pushing the shared portion 13g based on a translational displacement; thereby the holding finger 13 is configured so as to grasp/release an object using the V-shaped sections 13v.

The finger base 11, shaped like a plane-washer, comprises a hole "11e" bored at the center thereof; two protruded portions "11t" on the outer circumference; and two notches "11c" that engage the finger base with the holding finger 13, by clamping the end portion of the V-shaped sections 13v, i.e., the one end portion of the V-shaped sections 13v. The protruded portions 11t comprise slits "11s" and arc-shaped slits "11g" that communicate with the slits 11s, thereby a spring resilience is generated by the slits 11g in the direction to expand the slits 11s, if the side facets of the protruded portions 11t are pressured and held. Therefore, the finger base 11 is configured in such a way that the side facets of the protruded portions 11t are pressured and held so as to be inserted into grooves of a finger holder "62", resultantly the protruded portions 11t are clamped to the grooves by means of the spring resilience by releasing the holding.

Moreover, although the slits 11g are formed with a predefined length in an arc on the left and right sides of the slits 11s, the slits may be formed on only one of the sides, as far as a pressed spring resilience acts on the protruded portions 11t.

The conversion mechanism 30 is connected to the shaft 52a of the motor 52, and comprises a cam "33" and a cam follower "35" as a shifting element. As illustrated in Fig. 3, on the cam 33 is formed a cam face "33r" at one end facet of a stepped cam shaft and a rotation-prohibiting hole "33w", in the same shape as the motor shaft, at the center thereof, which fixedly receives the shaft 52a of the motor. The cam face 33r is comprised of a highest lift portion "33s", a lowest lift portion "33t", as lift portions that consist of two planes, and transitional portions "33u" and "33v" that connect those lift portions.

On the other hand, the cam follower 35 consists of three stepped cylinder portions "35a", "35b", and "35c", and a hole "35d" is bored on one end facet of the cylinder portion 35a in such a way that only a portion of the hole is included in the 35a. A parallel pin "37" is inserted into the hole 35d in such a way that the top and a part of the side facet of the pin protrude from the hole 35d. The parallel pin 37 and the cam face 33r of the cam 35 contacts each other at a portion, which becomes a contact portion "37y". The top of the parallel pin 37 is inserted into a groove "62u" of the finger holder 62. A screw hole "35e" is bored on the cylinder portion 35c, as one end portion of the cam follower, to which the shared portion 13g of the holding finger 13 is screwed.

A first coil spring "21" and a second coil spring "22" are fitted on the outer circumferences of the cam 33 and the cam follower 35. The second coil spring 22 is fitted, in a compressed state, between a stepped portion of the cam 33 and a motor case "66", and the first coil spring 21, as a pressuring member, is also fitted, in a compressed state, between the cylinder portion 35a of the cam follower 35 and a cylindrical hollow portion "62a" of the finger holder 62. In addition, the compressed force by the first coil spring 21 is adjusted to be greater than the compressed force by the second coil spring 22. The reason for this is to make the cam 33 and the cam follower 35 firmly contact each other.

The driving unit 50 is comprised of the motor 52 and a battery "53" that is a driving source for the motor. The motor 52 is provided with a speed reducer with a planetary gear mechanism. The first reason for using the motor having the speed reducer is that a rotational velocity can be reduced to a suitable velocity so as to optimize opening/closing velocities of the holding finger 13 by adjusting a shifting velocity of the translational displacement at the shared portion 13g of the holding finger 13. The second reason for using the motor is that the holding finger 13 is prevented from closing and then opening by maintaining in a stopping state a rotation of the motor 52 , even if power to the motor 52 is cut off by a switch "57" while the holding finger 13 holds an object.

The switch 57, as a switching means, is fixed to the case 60, and is used for staring/stopping the motor 52 by connecting the battery 53, as a DC power source, to the motor 52 or disconnecting the battery from the motor; thereby the switch 57 is configured so as to rotate/stop the motor 52 in one direction with the same polarity of the battery 53.

The case 60 houses the conversion mechanism 30, and comprises: the finger holder 62, as a first case member, for fixing the finger base 11 of the finger unit 10; the motor case 66, as a second case member, for fixing one end portion of the motor 52 and one end portion of the finger holder 62; a driving case "64" for housing the battery 53; and a ring "70", as an elastic member as well as an adjusting member made of rubber having a hollow portion, that is placed between the finger holder 62 and the motor case 66.

That is, one end portion of the ring 70 is connected to the holding finger 13, and the other end portion is connected to the motor case 66. Moreover, a male screw "62n" is threaded on the outer circumference of the finger holder 62, and a female screw, which is connected to the male thread 62n, is threaded along the inside face of the one end portion of the motor case 66, and the finger holder 62 and the motor case 66 are screwed together.

Here, the ring acts, as a pressuring means, by adjusting the screwed amount between the finger holder 62 and the motor case 66, and the translational displacement position of the one end portion of the holding finger 13 is varied in accordance with the variation of an elastically deformed amount of the pressured ring 70. Therefore, because the distance between the one end portion and the other end portion of the holding finger 13 in a translational displacement direction is decreased, an opening/closing amount of the finger 13 can be adjusted.

In Fig. 4, in the finger holder 62, the cylindrical hollow portion 62a is formed and also a hole "62e" is bored at the center thereof, into which the cylinder portion 35c of the cam follower 35 is inserted. And, the grooves 62u with semicircle section, along which the parallel pin 37 of the cam follower 35 is slid, is cut inside the cylindrical hollow portion 62a along the translational displacement direction, whose length is longer than the translational displacement. Here, the reason why the parallel pin 37 of the cam follower 35 is slid and shifted in the groove 62u of the finger holder 62 is to prevent a rotational movement of the cam follower 35 due to a rotational movement of the cam 33. On other hand, six slits "67", i.e. there pairs of slits, are cut along the diameter of the finger holder 62 at the tip of the cylinder. Here, the reason why the multiple slits are provided is to select any slits with respect to the position of the switch 57.

The widths of the six slits 67 each are slightly narrower than the width of the protruded portions 11t, and when the protruded portions 11t of the finger base 11 are engaged to one of the pairs of the slits 67, the finger base 11 is fixed to the slits 67 with a predefined spring force. After the finger base 11 is installed, the shared portion 13g of the holding finger 13 is fixed by the fixing screw 15 to the screw hole 35e of the cam follower 35.

As described above, when the shared portion 13g of the holding finger 13 has been fixed with the fixing screw 15 to the screw hole 35e of the cam follower 35, the opening/closing amount adjusting mechanism of the holding finger 13 comprises: the finger holder 62 to which the finger unit 10 is connected; the motor case 66; and the ring 70.

Moreover, the first coil spring 21 and second coil spring 22 are configured in such a way that the total compressed force by both springs pushes the cylinder portion 35a of the cam follower 35 in the direction of an arrow "A" so that the cam face 33r of the cam 33 and the parallel pin 37 of the cam follower 35 abut each other at all times. The reason for being configured described above is to transmit the operation of the cam 33 by the rotation of the motor 52 to the cam follower 35 without fail.

The second coil spring 22 decreases a pressuring force on the shaft 52a of the motor 52 in a thrust direction, and decrease a burden of the shaft 52a by subtracting a thrust force, which is affected from the motor 52, from a pressuring force of the first coil spring 21 as a pressuring member.

The adjusting operation of an opening/closing amount of the holding finger 13 of the electric tweezers configured as described above, will be explained using Fig. 1. In a state where the tips of the holding finger 13 are opened up to its maximum amount, a predefined opening mount is selected by rotating the finger holder 62 with respect to the motor case 66. In other words, if the finger holder 62 is rotated in the direction of pressuring the ring 70, the opening mount is increased, and if the holder is rotated in the contrary direction, the opening mount is decreased.

In these cases, the opening/closing amount of the holding finger 13 is magnified as several dozen times as that of the shaft-directional displacement of the finger holder 62. Here, if a magnifying rate is 15, a translational displacement amount of 0.1 mm is needed in order to obtain an opening/closing amount of 1.5 mm at the tip of the holding finger 13. In this case, if the screw pitch of the finger holder 62 is 1 mm, this becomes equivalent to a rotational angle of 36° of the screw, calculated by the following formula. 360° × 0.1 = 36° That is, because the opening/closing amount of the holding finger 13 can be adjusted with a small rotational amount, and a translational displacement amount of 0.1 mm is sufficient in this case, any special rubber having large elastic deformation is not needed for the ring 70.

Next, when an operator pushes down the switch 57, the motor 52 starts to rotate in accordance with a voltage applied from the battery 53 to the motor 52. Thereby, the cam 33, in response to this rotation, starts to rotate in the same direction. When the parallel pin 37 of the cam follower 35 is moved from the transitional portion 33v to the lowest lift portion 33t, and the switch 57 is shut off, the shared portion 13g of the holding finger 13 is pulled in the arrow A direction so that the holding finger 13 is closed with its maximum amount to hold an object.

At this time, although the holding finger 13 tries to return to its opening state by a spring resilience, a reverse rotation is prevented by a frictional force on the reducing gear of the motor 52. At a result, the closing state of the V-shaped sections 13v of the holding finger 13 is maintained, so that the finger continues to hold the object. And now, the closing amount of the holding finger 13 is determined based on a lifting amount of the cam face 33r by referring to the maximum opening amount. On the other hand, when the operator pushes down the switch 57 again, the motor further rotates to contact the transitional portion 33u of the parallel pin 37 of the cam follower 35 with the highest lift portion 33s. Thereby, the shared portion 13g of the holding finger 13 is shifted back in the direction indicated by an arrow "B", and the object is released.

In the electric tweezers 1 in this Embodiment, the opening/closing amount of the holding finger can be set in advance to a predefined amount at holding work. Therefore, the operator not only can be released from using finger force to hold the object, such as parts, by the hold finger 13, but also can exert the optimal force to the object. Thereby, a particular skill is not needed for work of holding a brittle object, etc.

Moreover in this Embodiment, although the ring 70 is inserted between the finger holder 62 and the case 66, an elastic element having suitable rigidity, such as a plane washer or a rubber washer, may be inserted.

### Embodiment 2

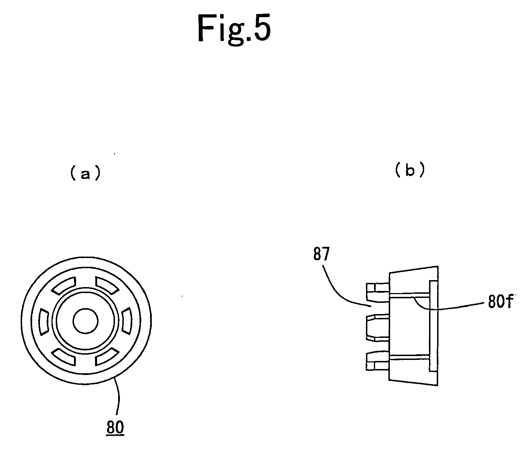

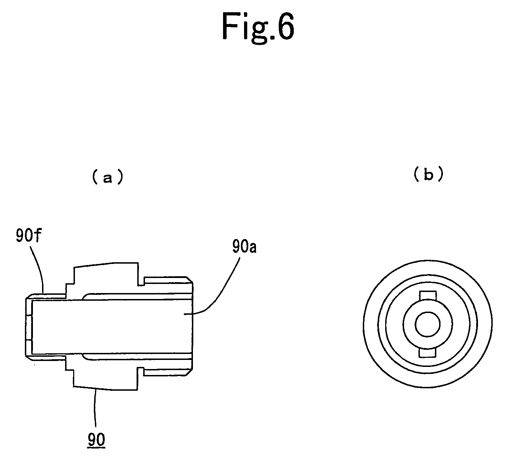

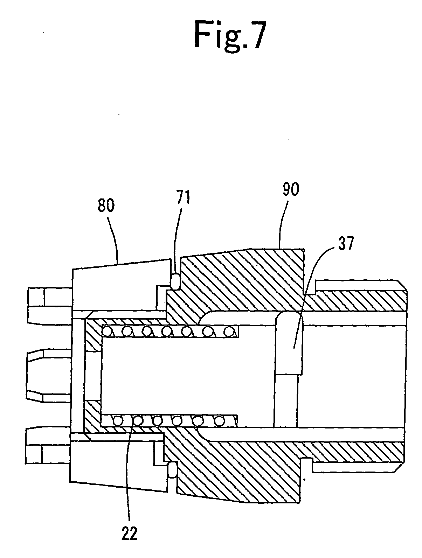

Another Embodiment of the invention will be explained using Fig. 5 through Fig. 7. Figs. 5 are views including a left side view "a" and an elevation view "b" of a finger holder used for electric tweezers according to another Embodiment of the invention; Figs. 6 are views including a left side view "a" and an elevation view "b" of a cam follower holder used for the electric tweezers according to another Embodiment of the invention; and Fig. 7 is a cross-sectional view of an assembly of the finger holder illustrated in Fig. 6 and the cam follower holder illustrated in Fig. 6.

The opening/closing amount adjusting mechanism of the holding finger 13 according to Embodiment 1 elastically has deformed the ring 70 by screwing the finger holder 62 into the motor case 66, so as to adjust an opening amount of the holding finger 13 by adjusting the position of one end portion of the holding finger 13.

However, because the first coil spring 21 is slightly contracted and expanded by deforming the ring 70 elastically, the holding force of the holding finger 13 is slightly varied.

Therefore, electric tweezers having an opening/closing amount adjusting mechanism is provided in this Embodiment, in which the contraction/expansion of the first coil spring 21 is not affected by the elastic deformation of the ring 70 as with above described Embodiment 1, and the holding force of the holding finger 13 is maintained at a constant value while the opening/closing amount of the holding finger 13 is adjusted.

In the opening/closing amount adjusting mechanism in Fig. 5 and Fig. 6, the finger holder 62 in Embodiment 1 is separated into two components that are a finger holder "80" (a first case member in Claim 5) and a cam follower holder "90" (a second case member in Claim 5).

The finger holder 80 comprises a hole bored at the center and multiple pairs of slits "87" in a diameter direction similar to the finger holder 62 in Fig. 4, and a screw "80f" to which the tip of the cam follower holder 90 is connected is threaded inside the hole, and the holder is connected and fixed to one end portion of the holding finger 13 by fixedly engaging the protruded portions 11t of the finger unit 10 with the slits 87 of the finger holder 80.

In the cam follower holder 90, a portion corresponding to the finger holder 80 is removed from the finger holder 62 in Fig. 4, and a screw "90f", on which the screw 80f of the finger holder 80 is screwed, is threaded, in place of the portion, outside a cylinder hollow portion "90a". Here, the finger holder 80 and the cam follower holder 90 functions as a pressuring means by adjusting a screwed amount.

In Fig. 7, the finger holder 80 is screwed to the cam follower holder 90 through a ring "71", as a first elastic as well as an adjusting member, and the cam follower holder 90 is formed in such a way that elastic deformation of the ring 70 does not affect the contraction/expansion of the first coil spring 21 by housing the cam follower 35 and the first coil spring 21 in the cam follower holder.

Moreover, the ring 71 is assembled in a state in which the ring is slightly elastically deformed.

On adjusting an opening/closing amount of the holding finger 13, a predefined opening amount is selected by rotating the finger holder 80 with respect to the cam follower holder 90, in a state where the tips of the holding finger 13 are opened at its maximum. That is, if the finger holder 80 is rotated in the direction of pressuring the ring 71, an opening mount is increased, and if the holder is rotated in the contrary direction, the opening mount is decreased.

In addition, the operation of the electric tweezers is omitted, because the operation is the same as that in Embodiment 1.

### Embodiment 3

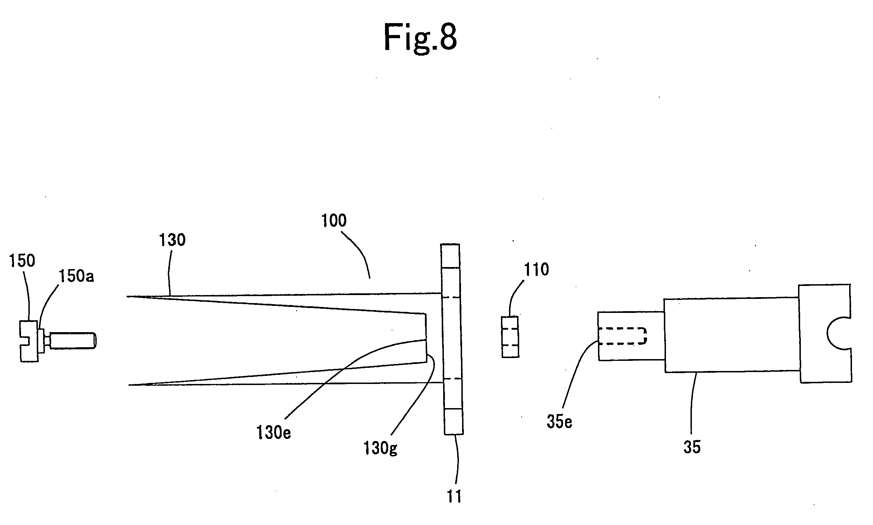

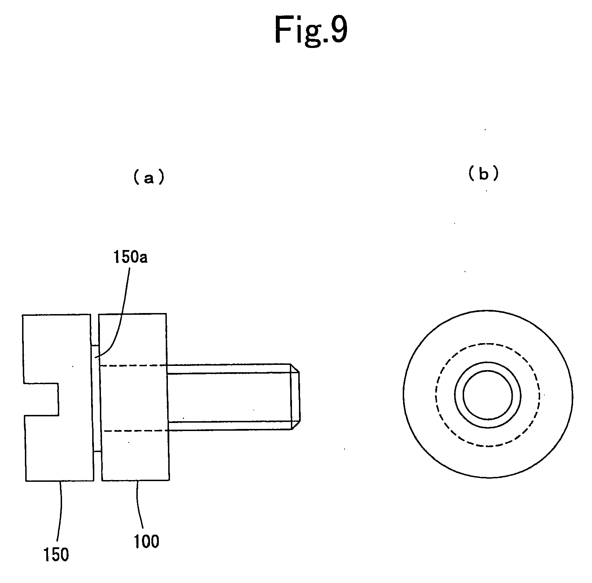

Another Embodiment of the invention will be explained using Fig. 8 through Fig. 9. Figs. 8 are exploded oblique perspective views of a opening/closing amount adjusting mechanism of the holding finger of the electric tweezers according to another Embodiment of the invention; and Figs. 9 are views including a left side view "a" and an elevation view "b" in which a fixing screw and an elastic washer illustrated in Fig. 8 are assembled together.

In Embodiment 1 and Embodiment 2 described above, although the opening/closing adjustment of the holding finger 13 is adjusted by adjusting a fixed position of the one end portion of the holding finger 13, a fixed position of a shared portion "130g" of a holding finger "130" is adjusted in this Embodiment.

In Fig. 8, the opening/closing amount adjusting mechanism is fixed by screwing, via an elastic washer "110", a fixing screw "150 into a screw hole "35e", at the center of the shared portion 130g of the holding finger 130, that is thread at one end portion of the cam follower 35, in which the elastic washer "110", being made of a material, such as nitrile rubber or ethylene rubber is a plane washer having a hollow portion, as the second elastic as well as adjusting member. Here, screwing the fixing screw 150 into the screw hole 35e provides the same function as that of the pressuring means as described above.

The fixing screw 150 is a screw member that has a stepped portion "150a", whose length (thickness) slightly greater than the thickness of the holding finger 130, and whose external diameter is slightly smaller than the internal diameter of a hole "130e" of the central shared portion 130g. Thereby, the central shared portion 130g of the holding finger 130 is loosely inserted into the stepped portion 150a which lies between the elastic washer 110 and the fixing screw 150. Therefore, even if the fixing screw 150 is rotated, the shared portion of the holding finger 130 is not twisted according to the rotation.

Moreover, the configuration, in which the protruded portions 11t of the finger base 11 of the finger unit "100" are engaged into the slits 67 of the finger holder 62, is the same as that in Embodiment 1.

The adjusting operation of an opening/closing amount of the holding finger 130 of the electric tweezers, as described above, will be explained. When the holding finger 130 is opened at its maximum, a predefined opening mount is selected by rotating the fixing screw 150. That is, if the fixing screw 150 is rotated in the direction of pressuring the elastic washer 110, an opening mount is increased, and if the holder is rotated in the contrary direction, the opening mount is decreased.

In the mean time, the operation of the electric tweezers is omitted, because the operation is the same as in Embodiment 1.

### Embodiment 4

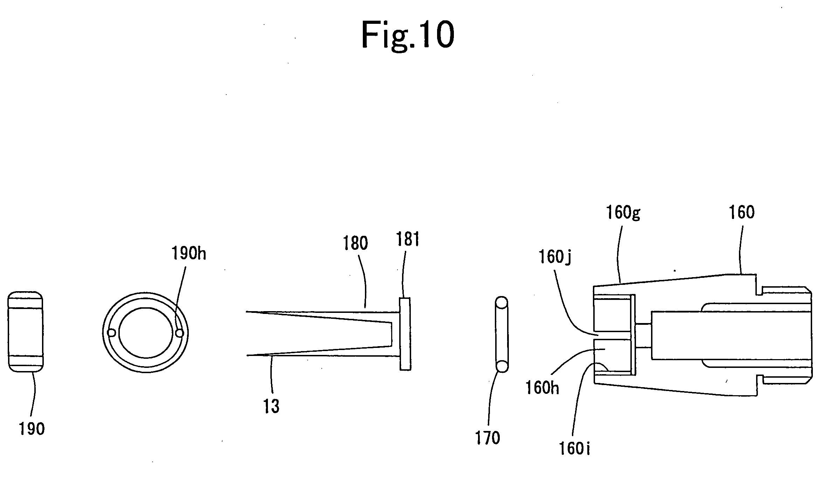

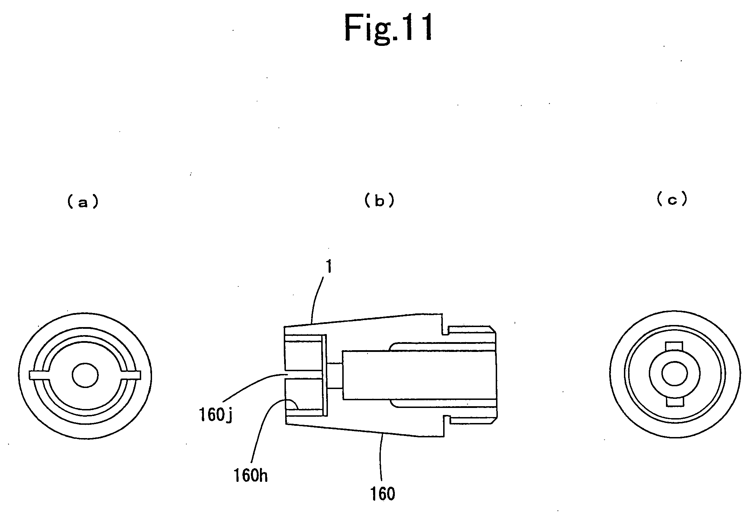

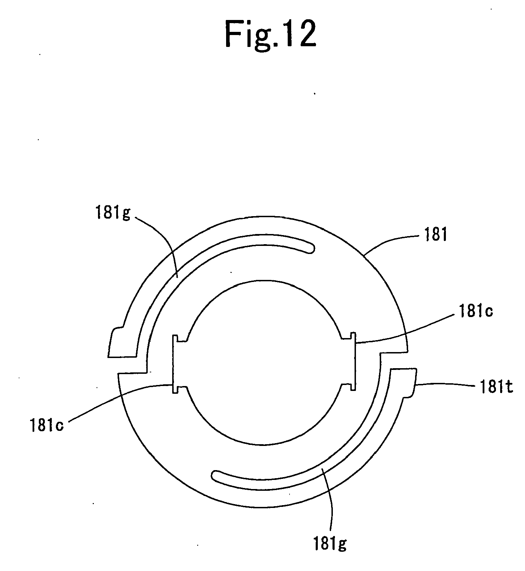

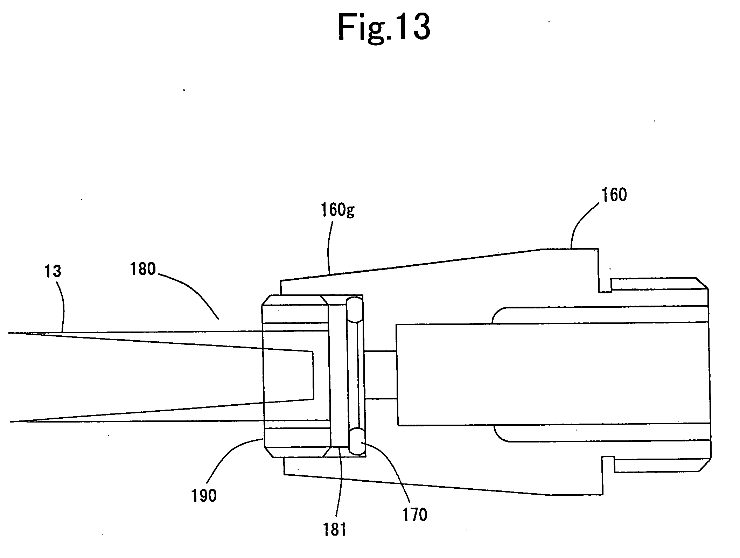

Another Embodiment of the invention will be explained using Fig. 10 through Fig. 13. Figs. 10 are exploded oblique perspective views of an opening/closing amount adjusting mechanism of a holding finger of electric tweezers according to another Embodiment of the invention; Figs. 11 are views including a left side view "a", an elevation view "b", and a right side view "c" of a finger holder used for the finger holder illustrated in Fig. 10; Fig. 12 is a plane view illustrating a finger base illustrated in Fig. 10; and Fig. 13 is a cross-sectional view of an assembly of the finger holder illustrated in Fig. 11 and the finger base, etc. illustrated in Fig. 12.

In this Embodiment, a modified example of Embodiment 2 is presented, and electric tweezers of which configuration is slightly simpler than the configuration in Embodiment 2, will be provided.

In Fig. 10 and Fig. 11, the opening/closing amount adjusting mechanism of the holding finger 13 includes: a finger holder "160" as a holding member, to which a finger unit "180" is fixed via a ring "170" having a hole, as a third elastic member and adjusting member, and that has a screw "160i" inside a hollow portion "160h"; and a pressuring nut "190" as a nut member, wherein a screw connected to the screw 160i is threaded outside the member, and which adjusts a opening/closing amount of the holding finger 13 by pressuring the finger unit 180 and by deforming the ring 170 elastically. Here, the mechanism functions operated as a pressuring means by screwing the pressuring nut 190 into the screw 160i of the finger holder 160.

The finger holder 160 is provided with a tip cylinder portion "160g" at the tip of the holder, and slits "160j" are formed in the diameter direction at the tip cylinder portion 160g. Other configurations other than that are the same as those of the Finger holder 62 illustrated in Fig. 4.

In Fig. 12, a finger base "181" (a base component), shaped like plane-washer, comprises a hole "181e" bored at the center thereof; two protruded portions "181t" on the outer circumference; and two notches "181c" that engage the finger base with the holding finger 13, by clamping the end portion of the V-shaped sections 13v.

The protruded portions 181t are separated with a predefined distance from the outer circumference, thereby a spring resilience is generated by arc-shaped slits "181g" in the direction to open the slits 181s, if the side facets of the protruded portions 181t are pressured and held. Thereby, the finger base 11 is configured in such a way that the side facets of the protruded portions 11t are pressured and held so as to be inserted into a concave portion of the finger holder 160, resultantly the protruded portions 11t are clamped to the concave portion by means of the spring resilience by releasing the holding.

Moreover, two holes "190h" are bored in the pressuring nut 190.

The adjusting operation of an opening/closing amount of the holding finger 13 of the electric tweezers, as described above, will be explained. In a state where the holding finger 13 is opened at its maximum, a rod-like members whose tip can be inserted into the holes 190h are at first inserted into the holes 190h of the pressuring nut 190, and a predefined opening amount is selected by rotating the pressuring nut 190. In other words, if the pressuring nut 190 is slightly rotated in the direction of pressuring the ring 170, an opening amount of the holding finger 13 is increased, and if the holder is rotated in the contrary direction, the opening amount is decreased.

Here, the operation of the electric tweezers is omitted, because the operation is the same as that in Embodiment 1.

When the finger unit 180 is replaced, the unit is removed by inserting a driver into the slits 160j of the finger holder 160 after the pressuring nut 190 has been removed.

### INDUSTRICAL APPLICABILITY

As described above, the electric tweezers related to the invention is suitable for holding and releasing an object.

## Claims

1. Electric tweezers comprising:
a motor having a shaft;
a conversion mechanism connected to the shaft, having a shifting element that in shifting converts unidirectional rotation of the shaft into predefined reciprocating translational displacement with respect to the shaft;
a case for housing the conversion mechanism and the motor;
a holding member, one end portion thereof being connected and fixed to the case, and the other end portion thereof being connected to the shifting element, for magnifying the amount by which the tweezer tips open/close based on the translational displacement of the shifting element, and for thereby releasing/holding an object;
a switching means for starting/stopping the motor rotation;
an adjusting member, one end facet thereof being connected either to the one end portion or the other end portion of the holding member, and the other end facet thereof being connected either to the shifting element or the case, so as to be elastically deformed; and
a pressuring means for pressuring the adjusting member a predefined amount, so as to regulate the separation between the one end portion and the other end portion in the translational displacement direction.

2. Electric tweezers comprising:
a motor having a shaft;
a conversion mechanism connected to the shaft, having a shifting element that in shifting converts unidirectional rotation of the shaft into predefined reciprocating translational displacement with respect to the shaft;
a case for housing the conversion mechanism, the case being divided into at least first and second case members, each member being threaded and the second case member are screwing into the first case member;
a holding member, one end portion thereof being connected and fixed to the first case member, and the other end portion thereof being connected to the shifting element of the conversion mechanism, for magnifying the amount by which, based on the translational displacement of the shifting element, the tweezer tips open/close, and for thereby releasing/holding an object;
a switching means for starting/stopping the motor rotation; and
a first elastic member having a hollow portion, the member being placed between the first case member and second case member, so as to be elastically deformed.

3. Electric tweezers comprising:
a motor having a shaft;
a case for housing the motor;
a holding member, one end portion thereof being connected and fixed to the case, and the other end portion thereof for magnifying the amount by which, based on reciprocating translational displacement, the tweezer tips open/close, and for thereby releasing/holding an object;
a switching means for starting/stopping the motor rotation;
a conversion mechanism, connected to the shaft, having a screw hole at one end of a shifting element that in shifting converts unidirectional rotation of the motor into predefined reciprocating translational displacement with respect to the other end of the holding member;
a screw member screwed to the screw hole; and
a second elastic member having a hollow portion, the member being placed between the screw member and the shifting element, so as to be elastically deformed.

4. Electric tweezers as recited in claim 2 or 3, wherein the conversion mechanism is connected to the shaft and further includes:
a cam having a lift portion;
a cam follower, connected to the other end portion of the holding member, for converting unidirectional rotation of the cam into predefined reciprocating translational displacement with respect to the other end portion, and having an abutting portion being abutted on the lift portion; and
a pressuring member housed in the case, for abutting, by the cam or the cam follower being pressured, the lift portion on the abutting portion.

5. Electric tweezers as recited in claim 4: wherein;
the pressuring member and the cam follower are housed in the second case member; and
the one end portion of the holding member is fixed to the first case member.

6. Electric tweezers comprising:
a motor having a shaft;
a conversion mechanism connected to the shaft, having a shifting element that in shifting converts unidirectional rotation of the shaft into predefined reciprocating translational displacement with respect to the shaft;
a holding member, having one end portion, the other end portion thereof being connected to the shifting element, for magnifying the amount by which, based on the translational displacement amount of the shifting element the tweezer tips open/close, and for thereby releasing/holding an object;
a base member having an engaging portion for engaging with the other end portion of the holder;
a finger holder having a hole, and a concave portion for engaging the base member attachably/detachably, the inside face of the concave portion being threaded;
a switching means for starting/stopping the motor rotation;
a conversion mechanism connected to the shaft, for converting a unidirectional rotation of the motor into predefined reciprocating translational displacement with respect to a shared portion of the holding member;
a third elastic member, having a hole, placed between the base member and the finger holder; and
a threaded nut member for screwing to the thread of the finger holder.

7. Electric tweezers as recited in claim 6, wherein the conversion mechanism is connected to the shaft and further includes:
a cam having a lift portion;
a cam follower connected to the other end portion of the holding member, for converting unidirectional rotation of the cam into predefined reciprocating translational displacement with respect to the other end portion, and having an abutting portion being abutted on the lift portion; and
a pressuring member housed in the case, for abutting, by the cam or the cam follower being pressured, the lift portion on the abutting portion.
